# EUROPÄISCHE PATENTSCHRIFT

(11) **EP 3 788 974 B1**
(45) Veröffentlichungstag und Bekanntmachung des Hinweises auf die Patenterteilung: **30.10.2024**
(21) Anmeldenummer: 19195658.0
(22) Anmeldetag: 05.09.2019
(51) Int. Cl.: A61B 18/14

(54) **ABLATIONSSONDE**
ABLATION PROBE
SONDE D'ABLATION

(43) Veröffentlichungstag der Anmeldung: 10.03.2021
(73) Patentinhaber: Erbe Elektromedizin GmbH, 72072 Tübingen (DE)
(72) Erfinder: Mayer, Volker, 72072 Tübingen (DE)
(74) Vertreter: Rüger Abel Patentanwälte PartGmbB

(56) Entgegenhaltungen:
- EP-A1- 2 474 340
- US-A- 5 899 898
- US-A1- 2003 171 742
- US-A1- 2003 195 503
- US-A1- 2014 228 831

## Beschreibung

Die Erfindung betrifft eine Ablationssonde, insbesondere eine HF-Ablationssonde mit innerer Kühlung, insbesondere eine solche Kathetersonde.

HF-Ablationssonden, insbesondere solche in Katheterform, haben an ihrem distalen Ende eine oder auch zwei Elektroden, die mit HF-Spannung beaufschlagt sind, um umliegendes Gewebe zu erhitzen, bzw. koagulieren bzw. zu ablatieren, wenn sie in ein entsprechendes Körperlumen, beispielsweise in die Leber oder Lunge eines Patienten, eingeführt sind. Um den gewünschten medizinischen Behandlungseffekt zu erzielen, muss die metallische Elektrode jedoch möglichst lange kühl bleiben, beispielsweise um das Gewebe möglichst großflächig zu erhitzen und um oberflächliche Verbrennungen des Gewebes zu verhindern. Deswegen enthalten solche Ablationssonden typischerweise einen Kanal, durch den einem Innenraum ein Kühlfluid zugeführt wird, das den Elektroden Wärme entzieht und diese ableitet.

Aus der US 6,939,350 B2 ist eine solche HF-Ablationssonde bekannt, bei der die Elektroden auf einem mehrlumigen Schlauch angeordnet sind. Dieser enthält Kanäle zum Zu- und Abführen von Kühlfluid, um die Elektroden zu kühlen. Zur Sicherstellung einer ausreichenden Wärmeabfuhr soll der Schlauch aus einem Kunststoff mit relativ hoher spezifischer Wärmeleitfähigkeit von wenigstens 0,8 W/m*K bestehen.

Eine ebensolche Sonde ist aus der WO 03/034932 A1 bekannt.

Zur Erhöhung der Wärmeleitfähigkeit von Kunststoff ist es aus verschiedenen Quellen, beispielsweise der DE 85 04 999 U1**,** der DE 29 45 607 A1 und der US 3,485,234 bekannt, in Kunststoffteile Metalleinlagen einzubauen.

Es hat sich jedoch gezeigt, dass die Erhöhung der Wärmeleitfähigkeit des Schlauchs nachteilige Effekte hat.

Aus der US 2014/0228831 A1 ist ein Herzkatheter zur Kryoanwendung bekannt. Der zu kühlende Teil des Herzkatheters weist lokal eine erhöhte Wärmeleitfähigkeit auf. Dazu sind in einem ersten Bereich der Schlauchwand des Herzkatheters wärmeisolierende Partikel und in einem zweiten Bereich wärmeleitende Partikel eingebettet. Zur lokalen Erhöhung der Wärmeleitfähigkeit können verschieden geformte Partikel in die Schlauchwand eingebettet sein. Ein so ausgebildeter wärmeleitender Bereich kann in Wärmeaustausch mit Elektroden stehen, um diese zu kühlen.

Weiter ist aus der US 2003/0195503 A1 ein Katheter mit Elektroden bekannt, die Taschen zur Aufnahme eines medizinisch wirksamen Stoffs aufweisen. Solche Taschen können die Katheterwand durchsetzen. Außerdem sind die Elektroden in die Schlauchwand eingebettet, sodass die Schlauchwand im Bereich der Elektroden dünner ist als im übrigen Bereich der Sonde.

Die US 5,899,898 offenbart eine innen gekühlte Sonde mit einer außenliegenden Elektrode. Zur Unterstützung der Kühlung der Elektrode kann dieselbe als Wendel ausgebildet sein, wobei diese Wendel die Schlauchwand mehrfach durchsetzt und somit eine thermische Verbindung vom Schlauchinneren nach außen schafft.

Davon ausgehend ist es Aufgabe der Erfindung, eine verbesserte Ablationssonde zu schaffen.

Diese Aufgabe wird mit der Ablationssonde gemäß Anspruch 1 gelöst:
Die erfindungsgemäße Sonde weist einen flexiblen Schlauch auf, der mindestens einen von einer Schlauchwand begrenzten Kanal aufweist und dessen Schlauchwand an einem distalen Endabschnitt mit wenigstens einer oder auch zwei oder mehreren Elektroden versehen ist. Die Schlauchwand weist in dem distalen Endabschnitt in radialer Richtung, d.h., in Richtung von der Elektrode zu dem Kanal, eine höhere radiale Wärmeleitfähigkeit auf, als außerhalb dieses distalen Endabschnitts. Der distale Endabschnitt des Schlauchs ist mit dem übrigen Schlauch über einen Adapter in Gestalt einer aus einem Metall ausgebildeten und elektrisch leitfähigen Hülse verbunden, wobei sich die Hülse einerseits in den Endabschnitt und andererseits in den übrigen Schlauch erstreckt, um diese fluiddicht zu verbinden. Der Leiter als auch die Elektrode sind mit der elektrisch leitfähigen Hülse elektrisch verbunden.

Durch die hohe radiale Wärmeleitfähigkeit in dem distalen Endabschnitt, an dem die mindestens eine Elektrode angebracht ist, wird die von der Elektrode ausgehende Wärme gut auf das Kühlmedium übertragen und wirksam abgeleitet. Andererseits wird durch die schlechtere Wärmeleitfähigkeit der Schlauchwand außerhalb des distalen Endabschnitts verhindert, dass Körperwärme des Patienten in den Kanal des Schlauchs eindringt und das Kühlfluid vorwärmt. Damit kann der außerhalb des distalen Endabschnitts liegende Bereich der Ablationssonde als Gegenstromwärmetauscher dienen, in dem das kalte aus dem distalen Endabschnitt herauskommende Kühlfluid das durch eine Fluidleitung, beispielsweise ein Kapillarrohr, zuströmende Kühlfluid kühlt. Die in diesem proximalen Abschnitt der Ablationssonde durch die geringe Wärmeleitfähigkeit des Materials der Schlauchwand gegebene thermische Isolierung der Sonde verhindert auch eine Schädigung von Körpergewebe oder Endoskop durch Kälte, wie sie auftreten könnte, wenn der flexible Schlauch über seine gesamte Länge eine erhöhte Wärmeleitfähigkeit aufwiese.

Die in dem distalen Endabschnitt erhöhte Wärmeleitfähigkeit der Schlauchwand kann durch verschiedene technische Maßnahmen herbeigeführt werden, die einzeln oder in Kombination miteinander angewendet werden können. Insbesondere kann die Schlauchwand in dem distalen Endabschnitt eine geringere radiale Dicke aufweisen als außerhalb des distalen Endabschnitts. Zusätzlich oder alternativ kann die Schlauchwand in dem distalen Endabschnitt ganz oder teilweise aus einem anderen Kunststoff bestehen als die übrige Schlauchwand, insbesondere aus einem Kunststoff mit höherer Wärmeleitfähigkeit.

Zusätzlich oder alternativ zu jeder der vorgenannten Maßnahmen kann die Schlauchwand in dem distalen Endabschnitt Wärmeübertragungskörper enthalten, die in die Schlauchwand eingebettet sind. Solche Wärmeübertragungskörper können Drähte, Bänder oder sonstige längliche Körper sein, die in Umfangsrichtung verlaufend angeordnet und beispielsweise als kurze Hülsen oder Ringe ausgebildet sind. Solche länglichen Wärmeübertragungskörper können auch einer Schraubenlinie folgend oder in Längsrichtung verlaufend angeordnet sein. Vorzugsweise bestehen sie aus einem wärmeleitfähigen Material, wie beispielsweise Kupfer, Silber, Aluminium oder auch Stahl, insbesondere Edelstahl oder Kohlenstofffasern. Alternativ können die Wärmeübertragungskörper unregelmäßig geformte und unregelmäßig angeordnete Partikel sein, beispielsweise Drahtstücke, Faserstücke, Partikel, wie zum Beispiel Diamantpartikel, Kohlenstoffpartikel und dergleichen.

Weitere Einzelheiten von Ausführungsformen der Erfindung sind Gegenstand der Zeichnung, der Beschreibung oder von Ansprüchen. Es zeigen:
Figur 1 eine Sonde mit einem sie speisenden Gerät, in schematischer Übersichtsdarstellung,
Figur 2 die Sonde, in einer ausschnittsweisen längsgeschnittenen Darstellung,
Figur 3 die Sonde nach Figur 2, in einer vergrößerten Ausschnittsdarstellung,
Figur 4 bis 10 weitere Ausführungsformen der Sonde jeweils, in längsgeschnittener ausschnittsweiser Prinzipdarstellung.

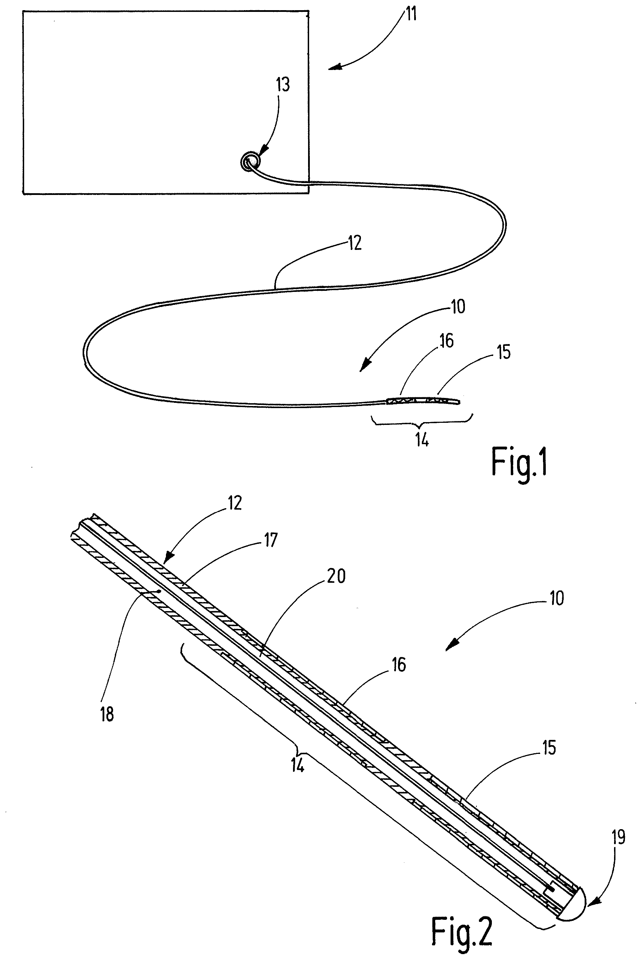

In Figur 1 ist eine Ablationssonde 10 veranschaulicht, die beispielsweise nach Art eines Katheters ausgebildet sein kann, um in Hohlgefäße eines Patienten, beispielweise ein Hohlgefäß der Lunge oder der Leber, eingeführt zu werden. Die Sonde 10 ist an ein speisendes Gerät 11 angeschlossen, das die Sonde 10 mit den erforderlichen Betriebsmedien speist, zu denen HF-Strom und ein Kühlfluid gehören können.

Die Sonde 10 besteht im Wesentlichen aus einem flexiblen Schlauch 12, der proximal zum Beispiel über einen geeigneten Stecker 13 an das Gerät 11 angeschlossen ist. Der Schlauch 12 trägt in einem distalen Endabschnitt 14 mindestens eine oder auch zwei Elektroden 15, 16, die elektrisch mit einem in dem Gerät 11 angeordneten HF-Generator verbunden sind.

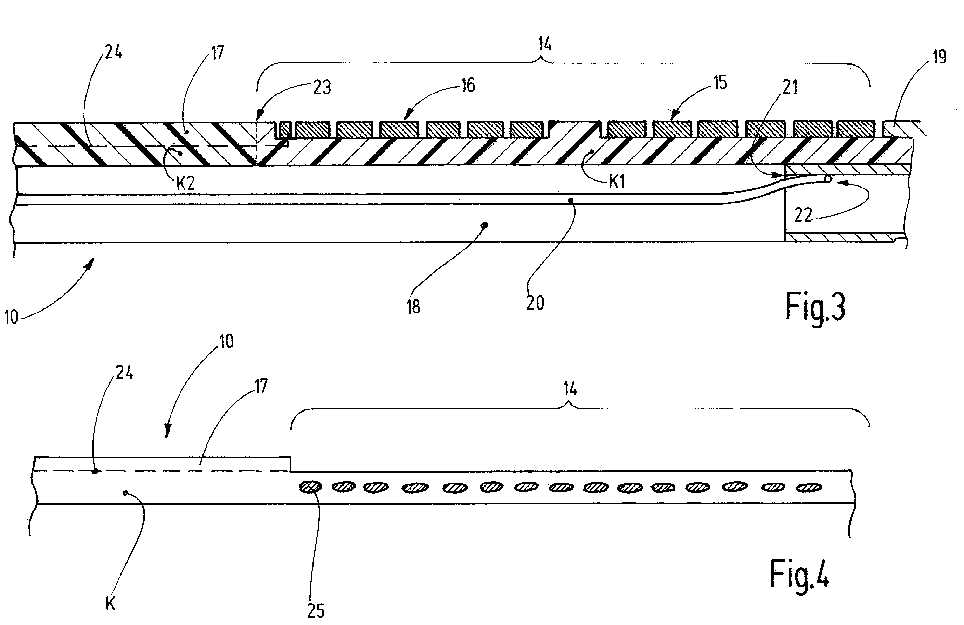

Der Aufbau der Sonde 10 insbesondere ihres distalen Endabschnitts 14 ist aus den Figuren 2 und 3 ersichtlich. Der Schlauch 12 weist eine Schlauchwand 17 auf, die einen Kanal 18 umschließt, der sich von dem proximalen Ende bis zu dem distalen Endabschnitt 14 der Sonde 10 und somit der Schlauchwand 17 erstreckt. Bei dem äußersten distalen Ende ist der Schlauch 12 durch ein Endstück 19 geschlossen. Das Endstück 19 kann elektrisch leitend ausgebildet sein, um die erste Elektrode 15 mit einer in dem Kanal 18 angeordneten durch ein dünnes Metallrohr gebildeten Fluidleitung 20 zu verbinden. Die Fluidleitung 20 kann ein Kapillarrohr beispielsweise aus Edelstahl oder einem anderen Metall sein, das, wie Figur 3 zeigt, endseitig über eine Schweißverbindung 21 mit einem Schaft des Endstücks 19 verbunden sein kann.

Die Fluidleitung 20 weist an ihrem mit dem Schaft des Endstücks 19 verbundenen Ende eine Düsenöffnung 22 auf, über die in Betrieb ein Kältemittel beispielsweise CO₂ ausgegeben wird, das dabei durch Expansion abkühlt und über den Kanal 18 zu dem Gerät 11 oder einer nicht weiter veranschaulichten Auslassöffnung der Sonde 10 zurückströmt.

Die Schlauchwand 17 besteht vorzugsweise aus einem Kunststoff, insbesondere einem schlecht wärmeleitenden Kunststoff, dessen Wärmeleitfähigkeit außerhalb des distalen Endabschnitts 14 gering, vorzugsweise geringer als 0,8 W/m*K ist. Im Bereich der beiden Elektroden 15, 16, d.h., in dem distalen Endabschnitt 14, weist die Schlauchwand 17 jedoch eine erhöhte Wärmeleitfähigkeit auf, sodass der Wärmewiderstand in radialer Richtung, d.h., von den Elektroden 15, 16 zu dem Lumen oder Kanal 18, größer als 0,8 W/m*K ist. Dazu kann der distale Endabschnitt der Schlauchwand 17 in dem distalen Endabschnitt 14 aus einem anderen, besser wärmeleitfähigen Kunststoffmaterial bestehen, als der übrige Teil der Schlauchwand 17. Die beiden verschiedenen Kunststoffmaterialien sind in Figur 3 symbolisch durch verschiedene Schraffuren veranschaulicht. An einer der Elektrode 16 proximal benachbarten Stelle 23 sind der den distalen Endabschnitt bildende Schlauchabschnitt und der übrige Schlauchabschnitt miteinander zum Beispiel durch Kleben oder Schweißen verbunden oder über ein zusätzliches kurzes dazwischenliegendes Adapter-Stück miteinander verkoppelt .

Sowohl der erste Kunststoff K1 des distalen Endabschnitts als auch der zweite Kunststoff K2 der sonstigen Schlauchwand 17 sind flexibel. Zum Erhalt der Flexibilität in dem distalen Endabschnitt 14 sind auch die Elektroden 15, 16 flexibel, beispielsweise indem diese durch ein schraubenförmig auf den Endabschnitt 14 des Schlauchs 12 gewickeltes Metallband gebildet sind. Während die erste Elektrode 15 elektrisch an die Fluidleitung 20 angeschlossen ist, kann zur Verbindung der zweiten Elektrode 16 mit dem Generator eine in der Schlauchwand 17 untergebrachte elektrische Leitung 24 vorgesehen sein. Diese kann beispielsweise ein sich in Axialrichtung erstreckender Leiter oder, wenn die Flexibilität erhöht werden soll, ein Leiter sein, der in der Schlauchwand 17 einer Schraubenlinie folgend angeordnet ist. Alternativ können eine oder beide Elektroden 15 und 16 auch über in dem Kanal 18 liegende Drähte kontaktiert werden, wobei hierzu ggf. entsprechende Durchbrüche in der Schlauchwand 17 erforderlich sind.

Die Wandstärke der Schlauchwand 17 kann im Bereich des Endabschnitts 14 reduziert sein, wie es in Figur 3 angedeutet ist. Alternativ kann sie auch mit der Wandstärke der übrigen Schlauchwand 17 übereinstimmend, d.h., ohne Dickenreduzierung ausgebildet sein. Indem die Schlauchwand 17 in dem Endabschnitt 14 aus einem Kunststoff K1 ausgebildet ist, dessen spezifische Wärmeleitfähigkeit (vorzugsweise deutlich) größer ist als die spezifische Wärmeleitfähigkeit des zweiten Kunststoffs K2 der übrigen Schlauchwand, werden die Elektroden 15, 16 bei geringem Kühlmittelverbrauch effizient gekühlt, wohingegen die Kälteeinwirkung entlang der Sonde 10 auf biologisches Gewebe oder das Endoskop außerhalb des distalen Endabschnitts 14 gering ist.

Die insoweit beschriebene Sonde 10 arbeitet wie folgt:
Im Betrieb ist die Sonde 10 in ein Körperlumen eines Patienten eingeführt. Befindet sich der distale Endabschnitt 14 mit den Elektroden 15, 16 an der zu behandelnden Stelle werden die Elektroden 15, 16 aktiviert, indem der Generator des Geräts 11 über die elektrische Leitung 24 und die elektrisch leitende Fluidleitung 20 eine HF-spannung zwischen den Elektroden 15, 16 anlegt. Von diesen geht ein Strom durch anliegendes biologisches Gewebe aus, das sich aufgrund seines ohmschen Widerstandes erwärmt und letztlich koaguliert wird. Dabei haben die Elektroden 15, 16 nassen Kontakt zu dem feuchten Gewebe.

Zugleich wird über die Fluidleitung 20 ein Kühlfluid, beispielsweise komprimiertes Kohlenstoffdioxid an der Düsenöffnung 22 abgegeben, von wo es sich in den Kanal 18 hinein entspannt. Es kann dabei eine adiabatische Abkühlung und gegebenenfalls durch den Joule-Thomson Effekt eine weitere Abkühlung erfahren, wobei Temperaturen von unter -40° erreicht werden können. Das somit zwischen den Elektroden 15, 16 und dem Kanal 18 aufgebaute Wärmegefälle führt zum Wärmefluss von den Elektroden 15, 16 in den Kanal 18, wodurch die Elektroden 15, 16 gekühlt werden. Diese bleiben dadurch feucht - eine Gewebeaustrocknung durch Verdampfen der Gewebeflüssigkeit wird vermieden.

Das noch kalte Kühlfluid strömt dann in den Kanal 18 in proximaler Richtung und somit dem in der Fluidleitung herangeführten Kühlfluid entgegen. Die Schlauchwand 17 isoliert diesen Kanal 18 dann in proximaler Richtung thermisch von der Umgebung, sodass die Fluidleitung 20 als effizienter Gegenstromwärmetauscher arbeitet und somit das Kühlfluid vorkühlt, bevor es zu der Düsenöffnung 22 gelangt.

An der insoweit beschriebenen Sonde sind zahlreiche Abwandlungen möglich. Beispielsweise kann die Sonde 10 lediglich eine einzige Elektrode 15 aufweisen, wobei der Stromkreis dann über eine externe, großflächig an dem Pateinten anzubringende Gegenelektrode geschlossen wird. Auch ist es möglich, an der Sonde 10 mehr als zwei Elektroden anzubringen, um beispielsweise Koagulation auf größerer Länge oder in einer zeitlich gestaffelten Abfolge vorzunehmen.

Weiter ist es möglich, die Schlauchwand 17 sowohl in dem distalen Endabschnitt als auch in dem übrigen Abschnitt aus einheitlichem Kunststoff auszubilden, d.h., die Kunststoffe K1 und K2 können gleich sein. In diesem Fall wird die erhöhte Wärmeleitfähigkeit der Schlauchwand 17 in dem distalen Endabschnitt durch eine Reduktion der Wandstärke an den Stellen erreicht, an denen die Elektroden 15, 16 angeordnet sind.

Die Kontaktierung der Elektroden 15 und 16 kann auch auf andere alternative Weisen erfolgen, als über die Fluidleitung 20 und einen elektrischen Leiter 24 in der Schlauchwand 17. Beispielsweise möglich ist eine Kontaktierung der Elektroden über Drähte oder Kabel in dem Kanal 18. Die Übergabe des Stromes von dem Lumen des Kanals auf die außenliegenden Elektroden ist dann beispielsweise über Durchbrüche in der Schlauchwand 17 zu den Elektroden oder Unterbrechungen der Schlauchwand durch elektrisch leitfähige Adapterstücke möglich. Die Fluidleitung 20 kann bei allen vor- und nachstehend beschriebenen Ausführungsformen auch mit mehr als einer Düse 22 ausgerüstet sein, wobei die Düsen 22 dann vorzugsweise als radial angeordnete Bohrungen im distalen Bereich der Fluidleitung ausgeführt sind.

Eine weitere beispielhafte Abwandlung zeigt Figur 4, wobei die vorstehende Beschreibung für die Ausführungsform nach Figur 4 mit Ausnahme der nachfolgend erläuterten Besonderheiten unter Zugrundlegung der bereits eingeführten Bezugszeichen entsprechend gilt. In dem distalen Endabschnitt 14 kann die Wandstärke der Schlauchwand 17 reduziert sein. Sie kann jedoch auch durchgängig gleichmäßig ohne Dickenreduzierung ausgeführt sein. In dem distalen Endabschnitt 14 ist ein Wärmeübertragungskörper 25 eingebettet, der im vorliegenden Ausführungsbeispiel durch einen in die Schlauchwand 17 eingebetteten einer Schraubenlinie folgenden Draht gebildet sein kann. Der schraubenförmige Wärmeübertragungskörper beschränkt sich dabei auf den distalen Endabschnitt 14. Die übrige Schlauchwand 17 enthält keinen solchen Wärmeübertragungskörper, sondern allenfalls den elektrischen Leiter 24. Bei monopolaren Anwendungen, die lediglich eine Elektrode aufweisen, kann der elektrische Leiter 24 entfallen.

Der Wärmeübertragungskörper 25 kann ein Runddraht, ein Profildraht, ein Flachdraht, ein Band oder dergleichen sein. Vorzugsweise besteht er aus einem gut wärmeleitfähigem Material wie zum Beispiel Kupfer, Silber, Aluminium oder auch einem immer noch ausreichend wärmeleitfähigen Material wie zum Beispiel Edelstahl. Unabhängig davon können anstelle eines schraubenförmig gewundenen Drahts beliebigen Profils auch ein oder mehrere Ringe vorgesehen sein, die axial nacheinander in einer Reihe in der Schlauchwand 17 angeordnet sind. Diese Ringe können einen beliebigen Ringquerschnitt zum Beispiel einen Rundquerschnitt, einen Rechteckquerschnitt oder einen anderweitig profilierten Querschnitt aufweisen. Der Wärmeübertragungskörper 25 besteht in diesem Fall aus mehreren Einzelkörpern, die voneinander distanziert angeordnet sind. Während sich der schraubenförmig ausgebildete Wärmeübertragungskörper 25 sowohl in Umfangsrichtung als auch in Axialrichtung erstreckt, sind die ringförmigen einzelnen Wärmeübertragungskörper ausschließlich in Umfangsrichtung orientiert.

Vorzugsweise ist der Wärmeübertragungskörper 25 jeglicher Bauform bei allen Ausführungsformen der Sonde 10 in der Schlauchwand 17 elektrisch isoliert und ohne Kontakt zu den Elektroden 15, 16 angeordnet.

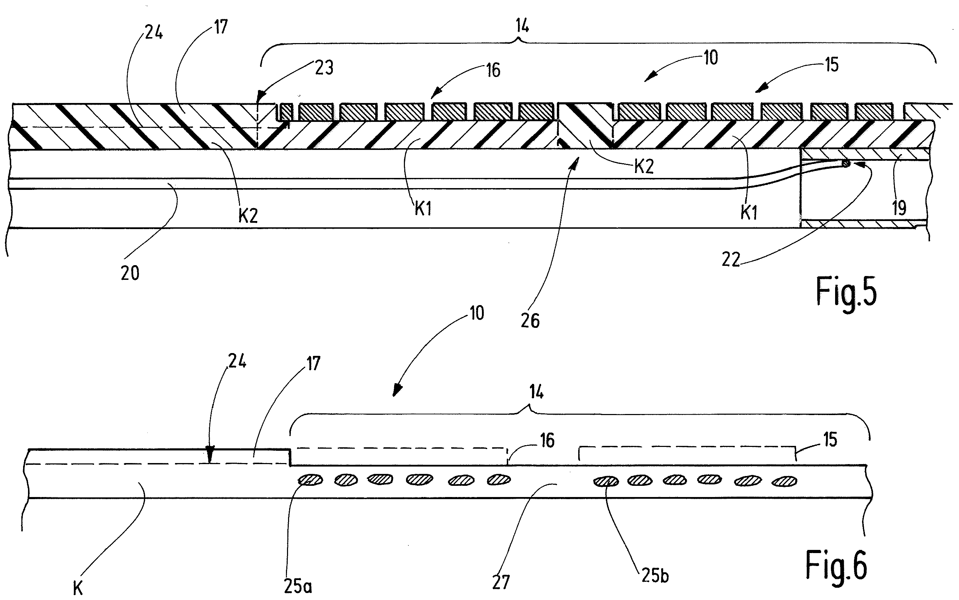

Eine weitere Ausführungsform der Sonde 10 geht aus Figur 5 hervor. Diese Ausführungsform beruht auf der Ausführungsform nach Figur 3, deren Beschreibung die Sonde nach Figur 5 unter Zugrundelegung der bereits eingeführten Bezugszeichen entsprechend gilt. Im Unterschied zu der Sonde 10 nach Figur 3 kann in dem distalen Endabschnitt 14 zwischen den beiden Elektroden 15, 16 jedoch noch ein Schlauchwandabschnitt 26 vorgesehen sein, der aus dem Kunststoff K2 des übrigen Schlauchs besteht, sodass in dem distalen Endabschnitt 14 lediglich die die Elektroden 15, 16 tragenden Schlauchwandabschnitte aus dem Kunststoff K1 bestehen. Dies verhindert zu starke Kälteeinwirkung auf biologisches Gewebe zwischen den beiden Elektroden 15, 16, was insbesondere Bedeutung hat, wenn der Schlauchwandzwischenabschnitt 26 eine nennenswerte axiale Länge aufweist, die zum Beispiel wenigstens so groß ist wie ein Viertel der axialen Länge einer Elektrode 15 oder 16.

Eine weitere Ausführungsform der Sonde 10 ist in Figur 6 veranschaulicht. Diese ist eine Abwandlung der in Figur 4 dargestellten Sonde 10, deren Beschreibung mit Ausnahme der nachfolgend erläuterten Besonderheiten entsprechend für die Sonde nach Figur 6 gilt.

Der Wärmeübertragungskörper 25 umfasst bei der Sonde nach Figur 6 zwei Wärmeübertragungsteilkörper 25a, 25b, die jeweils lediglich im Bereich der Elektroden 15, 16 angeordnet sind. In dem Zwischenbereich 27 der Schlauchwand 17 zwischen den beiden Elektroden 15, 16 fehlen Wärmeübertragungskörper. Ansonsten stimmt die Sonde 10 nach Figur 6 insbesondere hinsichtlich den Möglichkeiten der Gestaltung der Dicke der Schlauchwand 17 als auch hinsichtlich den möglichen Ausbildungen der Wärmeübertragungskörper entsprechend übertragen auf die Wärmeübertragungsteilkörper 25a, 25b mit der Sonde 10 nach Figur 4 überein.

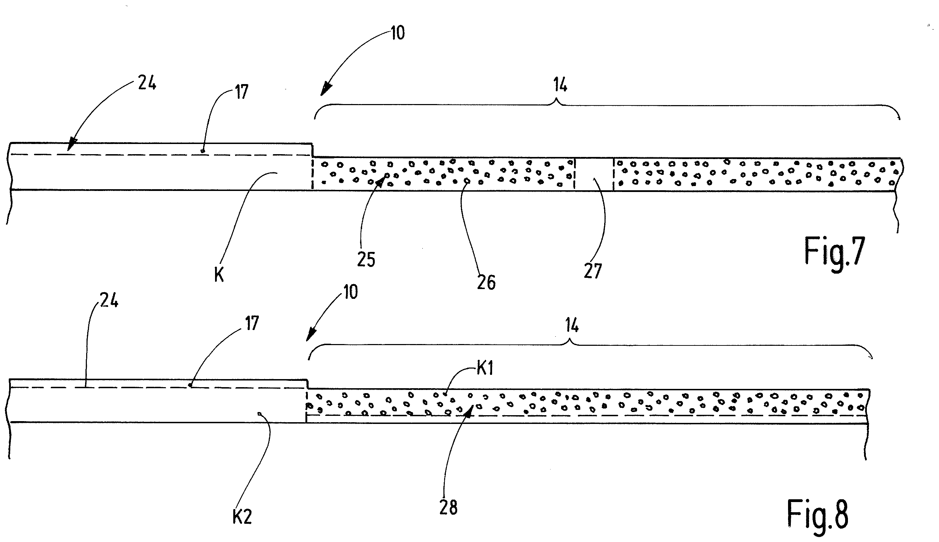

Figur 7 veranschaulicht eine Sonde 10, für die bis auf die Ausbildung der Wärmeübertragungskörper 25 die Beschreibung der Sonde 10 nach Figur 6 entsprechend gilt. Die Wärmeübertragungskörper 25 sind hier durch vielzählige, in die Kunststoffmatrix der Schlauchwand 17 eingebettete Partikel 28 gebildet. Diese bestehen aus einem wärmeleitfähigen Material, beispielsweise Metallpulver, Kohlenstoffpulver, Diamantpulver, Karbonfäden, Drahtstücken oder dergleichen. Der Zwischenbereich 27 zwischen den Elektroden 15, 16 ist frei von solchen Partikeln 28. Der Kunststoff des Endabschnitts 14 kann mit dem Kunststoff der übrigen Schlauchwand 17 übereinstimmen. Alternativ können die übrige Schlauchwand und der Zwischenbereich 27 aus einem Kunststoff K2 und die partikeltragenden Bereiche des Endabschnitts 14 aus dem Kunststoff K1 bestehen.

Figur 8 veranschaulicht eine Sonde 10, bei der die Schlauchwand 17 sowohl in dem Endabschnitt 14 als auch in dem übrigen Abschnitt aus ein- und demselben Kunststoff K besteht, wobei die Dicke der Schlauchwand 17 in dem Endabschnitt 14 zunächst stark reduziert ist. Auf diesen aus dem Kunststoff K2 bestehenden Teil der Schlauchwand 17 ist dann ein Mantel aus einem besser wärmeleitfähigen Kunststoffmaterial K1 aufgetragen, dessen spezifische Wärmeleitfähigkeit durch jede geeignete Maßnahme erhöht sein kann. Zum Beispiel kann der Kunststoff K1 ein intrinsisch besser wärmeleitfähiger Kunststoff sein. Alternativ kann seine Wärmeleitfähigkeit auch durch Einbettung von Wärmeübertragungskörpern 25 beispielsweise in Gestalt jedes der vorstehend beschriebenen Wärmeübertragungskörper 25 oder in Gestalt von Partikeln 28 erhöht sein. Hinsichtlich der Ausbildung der Elektroden sowie deren elektrischer Anschlüsse gelten für alle vorstehenden Ausführungsformen die im Zusammenhang mit den Figuren 1 bis 34 sowie Figur 5 beschriebenen Einzelheiten.

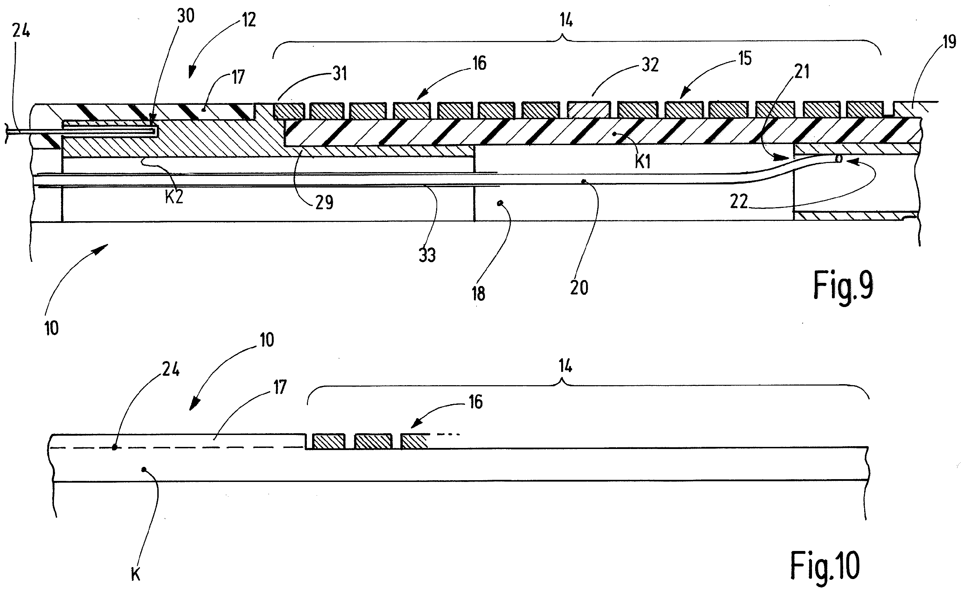

Figur 9 veranschaulicht eine erfindungsgemäße Ausführungsform der Sonde 10, bei der der Endabschnitt 14 aus einem dünnwandigen Schlauchabschnitt besteht, der über einen Adapter, z.B. in Gestalt einer Hülse 29 mit dem übrigen Schlauch 12 verbunden ist. Die Hülse 29 oder ein sonstiger Adapter erstreckt sich einerseits in den Endabschnitt 14 und andererseits in den übrigen Schlauch 12, um diese fluiddicht miteinander zu verbinden. Die Hülse ist aus einem Metall ausgebildet und elektrisch leitfähig. Sie kann aber auch aus einem anderen elektrisch leitfähigen oder auch aus einem elektrisch isolierenden Material ausgebildet sein, wie Keramik oder Kunststoff.

Der Schlauch 12 besteht aus dem Kunststoff K2. Der Endabschnitt 14 kann aus dem gleichen oder einem anderen Kunststoff K1 oder K2 bestehen. Dieser Kunststoff K2 kann die gleiche oder eine andere, insbesondere eine höhere spezifische Wärmeleitfähigkeit wie der Kunststoff K1 haben. Unabhängig davon, ob die spezifische Wärmeleitfähigkeit des Kunststoffs K1 größer, gleich oder kleiner als die spezifische Wärmeleitfähigkeit des Kunststoffs K2 ist, hat der Endabschnitt 14 aber, wenn er gegenüber dem Schlauch 12 eine geringeren Wandstärke aufweist, in Radialrichtung eine höhere Wärmeleitfähigkeit wie der Schlauch 12. Die Wandstärke des Endabschnitts 14 kann aber auch mit der Wandstärke des Schlauchs 12 übereinstimmen, wobei der Kunststoff K2 dann vorzugsweise eine höhere spezifische Wärmeleitfähigkeit aufweist als der Kunststoff K1. Die elektrische Kontaktierung der Elektrode 16 kann über die Hülse 29 erfolgen. Dazu sind sowohl der Leiter 24 als auch die Elektrode 16 mit der elektrisch leitfähigen Hülse 29 elektrisch verbunden. Die Leitung 24 kann in eine in der Wandung der Hülse 29 vorgesehene Längsbohrung 30 eingeführt und dort kontaktiert sein, z.B. durch eine Verformung der Hülse oder durch federnde Anlage des Leiters 24 an der Wandung der Längsbohrung. Alternativ kann der Leiter 24 auch an die Hülse 29 mittels Schweißen kontaktiert sein. Die Elektrode 16 kann an einer Kontaktstelle 31 eines Flansches der Hülse 29 an diese angeschlossen sein. Zwischen den Elektroden 15, 16 kann ein ringförmiger elektrisch isolierender Abstandshalter 32 angeordnet sein. Zur Vermeidung elektrischer Kurzschlüsse weist die Fluidleitung 20 wenigstens im Bereich der Hülse 29 eine elektrische Isolierung 33 auf.

Figur 10 veranschaulicht eine Ausführungsform der Sonde 10, bei der der Endabschnitt 14 in seinem Endabschnitt 14 eine reduzierte Wandstärke und dadurch eine erhöhte Wärmeleitfähigkeit aufweist. Im Übrigen gelten die zu den vorstehend erläuterten Ausführungsformen gegebenen Erläuterungen entsprechend.

Die erfindungsgemäße Sonde 10 ist insbesondere als HF-Ablationssonde einsetzbar und weist eine innere Kühlung auf, um die wenigstens eine Elektrode 15 am Gewebe feucht zu halten und eine übermäßige Erwärmung zu verhindern. Im Bereich der Elektrode 15 weist die Schlauchwand 17 des die Elektrode 15 tragenden Schlauchs eine erhöhte Wärmeleitfähigkeit auf, während sie im Übrigen außerhalb des elektrodentragenden distalen Endabschnitts 14 eine vergleichsweise geringere Wärmeleitfähigkeit aufweist. Die Erhöhung der Wärmeleitfähigkeit in dem distalen Endabschnitt 14 kann durch eine Reduktion der Wandstärke durch die Wahl eines geeigneten Kunststoffs durch Anordnung von Wärmeleitkörpern in der Schlauchwand oder durch eine Kombination von zwei oder mehreren dieser Merkmale erreicht werden. Maßgeschneiderte Kunststoffschläuche sind beispielsweise von der Firma Mikrolumen (www.mikrolumen.com) oder anderen Katheter-Fertigungs-Spezialisten erhältlich.

### Bezugszeichen:

- 10: Sonde
- 11: Gerät
- 12: Schlauch
- 13: Stecker
- 14: distaler Endabschnitt des Schlauchs 12
- 15: erste Elektrode
- 16: zweite Elektrode
- 17: Schlauchwand
- 18: Kanal / Lumen
- 19: Endstück
- 20: Fluidleitung
- 21: Schweißverbindung
- K1: Kunststoff des Endabschnitts 14
- K2: Kunststoff der Schlauchwand außerhalb des Endabschnitts 14
- K: Kunststoff der Schlauchwand
- 22: Düsenöffnung
- 23: Verbindungsstelle in der Schlauchwand 17
- 24: elektrischer Leiter
- 25: Wärmeübertragungskörper
- 25a, 25b: Wärmeübertragungsteilkörper
- 26: Schlauchwandzwischenabschnitt
- 27: Zwischenbereich
- 28: Partikel
- 29: Hülse
- 30: Längsbohrung
- 31: Kontaktstelle
- 32: Abstandshalter
- 33: elektrische Isolierung

## Patentansprüche

1. Sonde (10), insbesondere HF-Ablationssonde mit innerer Kühlung,
mit einem flexiblen Schlauch (12), der einen von einer Schlauchwand (17) begrenzten Kanal (18) aufweist und dessen Schlauchwand (17) an einem distalen Endabschnitt (14) mit wenigstens einer mit einem Leiter (24) verbundenen Elektrode (15) versehen ist,
wobei die Schlauchwand (17) in dem distalen Endabschnitt (14) in Richtung von der Elektrode (15) zu dem Kanal (18) eine höhere radiale Wärmeleitfähigkeit aufweist als außerhalb dieses distalen Endabschnitts (14),
**dadurch gekennzeichnet,**
**dass** der distale Endabschitt (14) des Schlauchs (12) mit dem übrigen Schlauch (12) über einen Adapter (29) in Gestalt einer aus einem Metall ausgebildeten und elektrisch leitfähigen Hülse verbunden ist, wobei sich die Hülse (29) einerseits in den Endabschnitt (14) und andererseits in den übrigen Schlauch (12) erstreckt, um diese fluiddicht miteinander zu verbinden, und
**dass** sowohl der Leiter (24) als auch die Elektrode (16) mit der elektrisch leitfähigen Hülse (29) elektrisch verbunden sind.

2. Sonde nach Anspruch 1, **dadurch gekennzeichnet, dass** die Schlauchwand (17) aus Kunststoff (K, K1, K2) ausgebildet ist.

3. Sonde nach Anspruch 2, **dadurch gekennzeichnet, dass** die Schlauchwand (17) in dem distalen Endabschnitt (14) aus einem ersten Kunststoff (K1) und ansonsten aus einem zweiten Kunststoff (K2) besteht.

4. Sonde nach Anspruch 3, **dadurch gekennzeichnet, dass** der erste Kunststoff (K1) einen geringeren spezifischen Wärmewiderstand als der zweite Kunststoff (K2) aufweist.

5. Sonde nach einem der vorstehenden Ansprüche, **dadurch gekennzeichnet, dass** die Schlauchwand (17) in dem distalen Endabschnitt (14) mindestens einen Wärmeübertragungskörper (25) aufweist.

6. Sonde nach Anspruch 5, **dadurch gekennzeichnet, dass** der mindestens eine Wärmeübertragungskörper (25) in die Schlauchwand (17) eingebettet angeordnet ist.

7. Sonde nach Anspruch 5 oder 6, **dadurch gekennzeichnet, dass** der mindestens eine Wärmeübertragungskörper (25) ein aus einem Metall bestehender Draht oder ein aus Metall bestehendes Band oder eine Gruppe von Ringen oder Hülsen ist.

8. Sonde nach einem der Ansprüche 5 bis 7, **dadurch gekennzeichnet, dass** der Wärmeübertragungskörper (25) sich in Umfangsrichtung und/oder in Längsrichtung erstreckend angeordnet ist.

9. Sonde nach einem der Ansprüche 5 bis 7, **dadurch gekennzeichnet, dass** mehrere Wärmeübertragungskörper (25a, 25b, 28) vorgesehen sind.

10. Sonde nach Anspruch 9, **dadurch gekennzeichnet, dass** die Wärmeübertragungskörper (28) unregelmäßig angeordnet sind und/oder unregelmäßig geformte Partikel sind.

11. Sonde nach einem der vorstehenden Ansprüche, **dadurch gekennzeichnet, dass** die Schlauchwand (17) an dem distalen Endabschnitt (14) eine Wandstärke aufweist, die geringer ist als die sonstige Wandstärke der Schlauchwand (17).

12. Sonde nach einem der vorstehenden Ansprüche, **dadurch gekennzeichnet, dass** in dem Kanal (18) eine Fluidleitung (20) angeordnet ist, die in dem distalen Endabschnitt (14) des Schlauchs (12) endet.

13. Sonde nach Anspruch 12, **dadurch gekennzeichnet, dass** die Fluidleitung (20) als metallisches Kapillarrohr ausgebildet ist.

## Claims

1. Probe (10), particularly radio frequency ablation probe with inner cooling,
having a flexible hose (12) that comprises a channel (18) limited by a hose wall (17) and the hose wall (17) thereof is provided with at least one electrode (15) at a distal end section (14), wherein the electrode (15) is connected with a conductor (24),
wherein the hose wall (17) has a higher radial heat conductivity in the distal end section (14) in direction from the electrode (15) to the channel (18) than outside of this distal end section (14),
**characterized in that** the distal end section (14) of the hose (12) is connected with the remaining hose (12) via an adapter (29) in the form of a sleeve made of a metal and being electrically conductive, wherein the sleeve (29) extends on one side in the end section (14) and on the other side in the remaining hose (12) in order to connect them in a fluid-tight manner, and
that the conductor (24) as well as the electrode (16) are electrically connected with the electrically conductive sleeve (29).

2. Probe according to claim 1, **characterized in that** the hose wall (17) is made of plastic (K, K1, K2).

3. Probe according to claim 2, **characterized in that** the hose wall (17) consists of a first plastic (K1) in the distal end section (14) and apart therefrom of a second plastic (K2) .

4. Probe according to claim 3, **characterized in that** the first plastic (K1) has a lower specific heat resistance than the plastic (K2).

5. Probe according to any of the preceding claims, **characterized in that** the hose wall (17) comprises at least one heat transfer body (25) in the distal end section (14).

6. Probe according to claim 5, **characterized in that** the at least one heat transfer body (25) is arranged in an embedded manner in the hose wall (17).

7. Probe according to claim 5 or 6, **characterized in that** the at least one heat transfer body (25) is a wire consisting of a metal or a band consisting of a metal or a group of rings or sleeves.

8. Probe according to any of the claims 5-7, **characterized in that** the heat transfer body (25) is arranged in a manner extending in circumferential direction and/or in longitudinal direction.

9. Probe according to any of the claims 5-7, **characterized in that** multiple heat transfer bodies (25a, 25b, 28) are provided.

10. Probe according to claim 9, **characterized in that** the heat transfer bodies (28) are irregularly arranged and/or are irregularly formed particles.

11. Probe according to any of the preceding claims, **characterized in that** the hose wall (17) has a wall thickness at the distal end (14) that is smaller than the other wall thickness of the hose wall (17).

12. Probe according to any of the preceding claims, **characterized in that** a fluid conduit (20) ending in the distal end section (14) of the hose (12) is arranged in the channel (18).

13. Probe according to claim 12, **characterized in that** the fluid conduit (20) is configured as metallic capillary tube.

## Revendications

1. Sonde (10), en particulier sonde d'ablation HF à refroidissement intérieur,
comprenant un tube souple (12) qui présente un conduit (18) délimité par une paroi de tube (17) et dont la paroi de tube (17) est pourvue, sur une partie d'extrémité distale (14), d'au moins une électrode (15) reliée à un conducteur (24),
la paroi de tube (17) présentant, dans la partie d'extrémité distale (14), dans la direction allant de l'électrode (15) vers le conduit (18), une conductivité thermique radiale plus élevée qu'à l'extérieur de cette partie d'extrémité distale (14),
**caractérisée en ce que**
la partie d'extrémité distale (14) du tube (12) est reliée au reste du tube (12) par l'intermédiaire d'un adaptateur (29) ayant la forme d'un manchon réalisé en métal et conducteur de l'électricité, le manchon (29) s'étendant sur un côté dans la partie d'extrémité (14) et sur l'autre côté dans le reste du tube (12), afin de relier ces éléments l'un à l'autre de manière étanche aux fluides, et
**en ce que** le conducteur (24) et l'électrode (16) sont tous deux reliés électriquement au manchon (29) conducteur de l'électricité.

2. Sonde selon la revendication 1, **caractérisée en ce que** la paroi de tube (17) et réalisée en matière plastique (K, K1, K2).

3. Sonde selon la revendication 2, **caractérisée en ce que** dans la partie d'extrémité distale (14), la paroi de tube (17) est réalisée à partir d'une première matière plastique (K1) et est constituée par ailleurs d'une deuxième matière plastique (K2).

4. Sonde selon la revendication 3, **caractérisée en ce que** la première matière plastique (K1) présente une résistance thermique spécifique qui est plus faible que celle de la deuxième matière plastique (K2).

5. Sonde selon une des revendications précédentes, **caractérisée en ce que** la paroi de tube (17) présente au moins un corps de transmission de chaleur (25) dans la partie d'extrémité distale (14).

6. Sonde selon la revendication 5, **caractérisée en ce que** le corps de transmission de chaleur (25), au nombre d'au moins un, est disposé en étant encastré dans la paroi de tube (17).

7. Sonde selon la revendication 5 ou 6, **caractérisée en ce que** le corps de transmission de chaleur (25), au nombre d'au moins un, est un fil en métal ou une bande en métal ou un groupe de bagues ou de manchons.

8. Sonde selon une des revendications 5 à 7, **caractérisée en ce que** le corps de transmission de chaleur (25) est disposé de manière à s'étendre dans la direction périphérique et/ou dans la direction longitudinale.

9. Sonde selon une des revendications 5 à 7, **caractérisée en ce que** plusieurs corps de transmission de chaleur (25a, 25b, 28) sont prévus.

10. Sonde selon la revendication 9, **caractérisée en ce que** les corps de transmission de chaleur (28) sont disposés de façon irrégulière et/ou sont des particules de forme irrégulière.

11. Sonde selon une des revendications précédentes, **caractérisée en ce que** la paroi de tube (17) présente, dans la partie d'extrémité distale (14), une épaisseur de paroi qui est inférieure au reste de l'épaisseur de paroi de la paroi de tube (17).

12. Sonde selon une des revendications précédentes, **caractérisée en ce que** dans le conduit (18), il est prévu un conduit de fluide (20) qui se termine dans la partie d'extrémité distale (14) du tube (12).

13. Sonde selon la revendication 12, **caractérisée en ce que** le conduit de fluide (20) est réalisé sous forme de tube capillaire métallique.
